Europäisches Patentamt

(19) European Patent Office

Office européen des brevets

(11) Publication number: **0 064 681**
**A2**

## (12) EUROPEAN PATENT APPLICATION

(21) Application number: **82103678.7**

(22) Date of filing: **29.04.82**

(51) Int. Cl.³: **C 12 N 15/00**
**C 12 P 21/00, C 12 N 1/20**
**//C12R1/19**

(30) Priority: **30.04.81 JP 64201/81**

(43) Date of publication of application:
**17.11.82 Bulletin 82/46**

(84) Designated Contracting States:
**DE FR GB**

(71) Applicant: **Juridical Foundation Japanese Foundation for Cancer Research**
**37-1, Kami-Ikebukuro 1-chome Toshima-ku**
**Tokyo(JP)**

(72) Inventor: **Sugano, Haruo**
**4-8-13, Minami-Ogikubo Suginami-ku**
**Tokyo(JP)**

(72) Inventor: **Taniguchi, Tadatsugu**
**Yuparesu Tagara 303 4-27-12, Tagara**
**Nerima-ku Tokyo(JP)**

(74) Representative: **Vossius Vossius Tauchner Heunemann Rauh**
**Siebertstrasse 4 P.O. Box 86 07 67**
**D-8000 München 86(DE)**

(54) Novel recombinant DNA.

(57) Disclosed is a novel recombinant DNA containing the tufB promoter of *Escherichia coli* and a gene coding for the biosynthesis of a eukaryotic protein such as human interferon $\beta_1$. Also disclosed is a process for producing a eukaryotic protein by culturing a microorganism carrying a plasmid containing the tufB promoter and a gene coding for such protein.

EP 0 064 681 A2

./...

Fig. 1

# VOSSIUS · VOSSIUS · TAUCHNER · HEUNEMANN · RAUH
## PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

0064681

Our Ref.: R 833 EP (Vo/fw)          April 29, 1982
Case:   296-EPC

Juridical Foundation, Japanese Foundation for Cancer
Research, Tokyo, Japan

## "NOVEL RECOMBINANT DNA"

### Background of The Invention

The present invention relates generally to a novel recombinant DNA and more specifically to a recombinant DNA containing a promoter of the tufB gene coding for protein elongation factor Tu of Escherichia coli, a novel recombinant DNA containing the promoter and a gene coding for the biosynthesis of a eukaryotic protein, and a process for producing the eukaryotic protein using the recombinant DNA.

For the industrial use of microorganisms, extensive studies and development have heretofore been made on mass production of a eukaryotic protein by introducing a gene coding for the biosynthesis of the protein into a microorganism. In gene recombination technology, the promoter is an important factor; and, there is a need for the development of suitable promoters. To this end, it has now been found that the promoter in the tufB gene of Escherichia coli is an excellent promoter which can promote the synthesis of eukaryotic proteins. Heretofore, there have been no reports of a tufB promoter being recovered from the tufB gene and combined with a gene coding for the synthesis of a eukaryotic protein for the production of the eukaryotic protein by the recombinant DNA.

Summary of the Invention

In accordance with the present invention novel recombinant DNAs are produced using a promoter derived from the tufB gene of Escherichia coli (hereinafter referred to as "tufB promoter"). According to one aspect of the invention, a process for producing eukaryotic protein comprises culturing a microorganism carrying a plasmid containing the tufB promoter of Escherichia coli and a gene coding for said eukaryotic protein and which is capable of producing said eukaryotic protein, in a nutrient medium until recoverable quantities of said protein are accumulated in the culture liquor and thereafter recovering said protein therefrom.

The composition of matter aspects of the invention includes recombinant DNAs containing the tufB promoter of Escherichia coli, including recombinants with a plasmid DNA, recombinants wherein a gene coding for the biosynthesis of eukaryotic protein is combined downstream of the promoter, and recombinants containing a gene coding for the biosynthesis of eukaryotic protein wherein a cleavage site for the restriction endonuclease Cla I is constructed upstream from the gene.

Brief Description of the Drawings

Fig. 1 is a flow chart for preparing plasmid pTuB1P-5 of the present invention, wherein the arrow ↓ refers to cleavage sites with restriction endonuclease. The arrow (1) shows deletion of Bgl II and Eco RI cleavage sites;

Fig. 2 is a flow chart for preparing plasmid pIFNβ-4, wherein the arrow ↓ refers to cleavage sites with restriction endonucleases and the presence of linkers;

Fig. 3 is a flow chart for preparing plasmid pTuIFNβ-5 wherein the arrow ↓ refers to cleavage sites with restriction endonucleases and the presence of linkers; and

Fig. 4 is an illustration of the base sequence of the combining sites of the tufB promoter, synthetic DNA and interferon $\beta_1$ cDNA.

Description of the Invention

The tufB gene of Escherichia coli is one of the genes known as a polypeptide chain elongation factor being produced massively by Escherichia coli [Gurdon, Biochemistry Vol. 9, pp. 912 - 917 (1970)]. The tufB gene is described in detail in Bacteriol. Rev. Vol. 40, pp. 116 - 167 (1976). Moreover, the tufB gene cloned in plasmid pTUBl of Escherichia coli is described in Miyajima et al., FEBS Letters Vol. 102, No. 2, pp. 207 - 210 (1979).

The tufB promoter is recovered from the tufB gene as follows. Plasmid pTUBl DNA of Escherichia coli which contains the tufB gene [Miyajima et al., FEBS Letters Vol. 102, No. 2, pp. 207 - 210 (1979)] is cleaved with Bgl II (A↓G), a restriction endonuclease recognizing AGATCT and described in Nucleic Acids Res., 3, 1747 (1976), and the resulting cohesive end is treated with a DNA polymerase to make a blunt end. The product is then cleaved with Cla I (T↓C), a restriction endonuclease recognizing ATCGAT produced by Boehringer Mannheim GmbH, and a tufB promoter fragment of about 1 kilobase (kb) is isolated by a known method, for example, the method described in Tabak and Flavell, Nucleic Acids Res., Vol. 5 pp. 2321 - 2332 (1978).

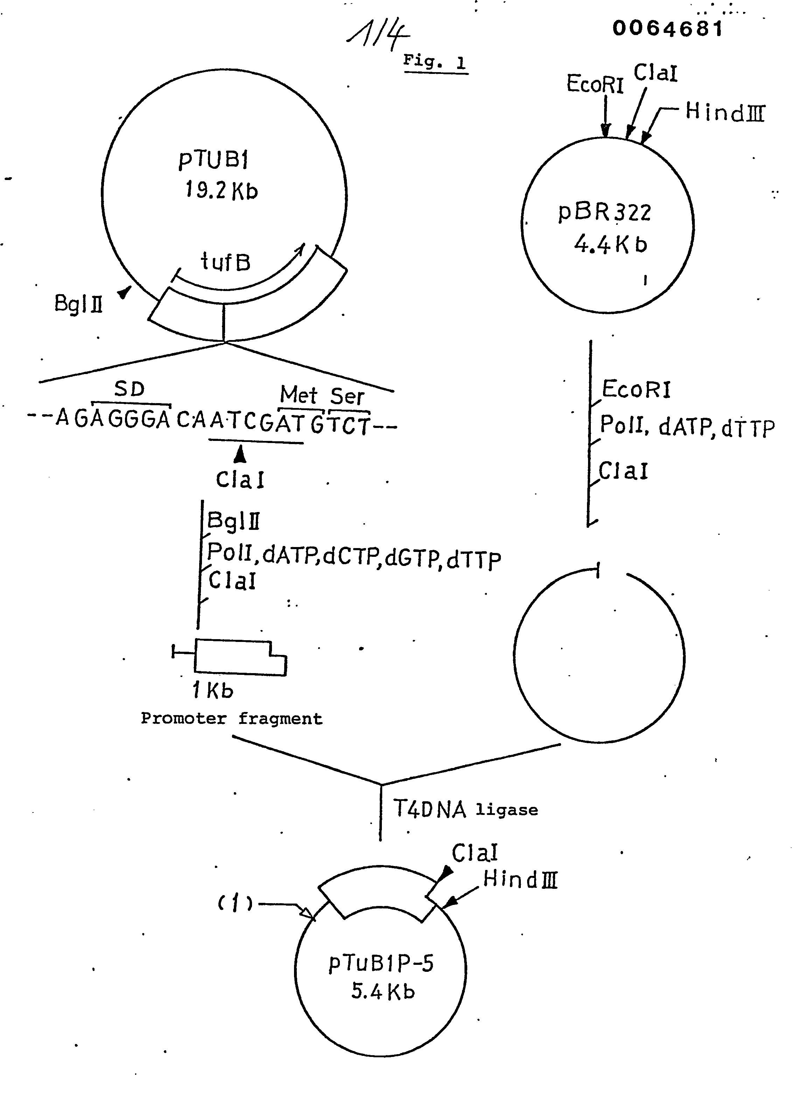

Separately, plasmid pBR 322 DNA [Gene, 2, 95 (1977)] is cleaved with Eco RI (G↓A), a restriction endonuclease recognizing GAATTC [Methods Mol. Biol., 7, 87 (1974)], and the resulting cohesive end is treated with a DNA polymerase to make a blunt end. The product is then cleaved again with Cla I. The resulting fragment and the promoter fragment described above are ligated with T4 DNA ligase by the method described in Weiss et al., J. Biol. Chem. 243, p. 4543 (1968). Thus, plasmid pTuBlP-5 containing the tufB promoter is obtained. Plasmid pTuBlP-5 is illustrated in Fig. 1. Escherichia coli HB101 containing pTuBlP-5 has been deposited with the American Type Culture Collection, U.S.A. under accession number ATCC 31878. Since pTuBlP-5 has only one cleavage site for Cla I, an objective gene can be conveniently constructed downstream from the tufB promoter.

The tufB promoter of the present invention can be used for the production of eukaryotic proteins by combination with a gene coding for the synthesis of eukaryotic proteins. As suitable eukaryotic proteins, any protein may be applied so long as the productivity of the protein is increased when a gene coding for the synthesis of the protein is recombined with upstream tufB promoter and a microorganism containing the recombinant DNA is cultured. Preferably, genes coding for human interferon, insulin, somatostatin and the like are used.

For the production of new recombinant DNAs of the present invention, a vector DNA is required. As the vector DNA, plasmid DNAs or phages derived from Escherichia coli may be used. Preferably, pBR 322, pMB 9, pCR 1 and the like are used. In the present invention, pBR 322 is most preferably used.

An example of the process for the production of the novel recombinant DNA of the present invention containing the tufB promoter and a gene coding for the synthesis of eukaryotic protein is described below using as a gene coding for human interferon $\beta_1$ and as a vector DNA plasmid pBR 322.

Initially, a process for the production of plasmid pIFNβ-4 which may be conveniently used to combine with the tufB promoter and which contains human interferon $\beta_1$ is described.

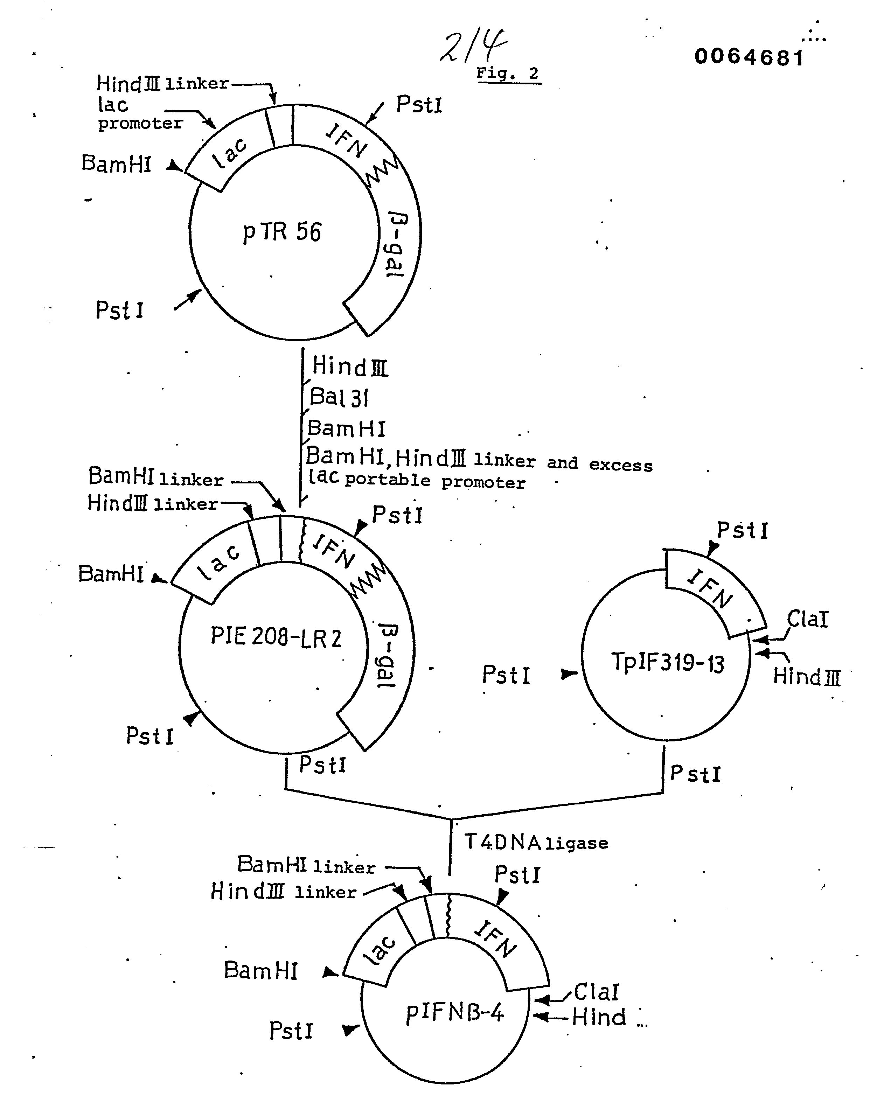

Plasmid pTR 56 containing a partially deleted human inteferon $\beta_1$ gene [Taniguchi et al., Proc. Natl. Acad. Sci. USA, Vol. 77, No. 9, pp. 5230 - 5233 (1980)] is cleaved with Hind III (A↓A) which is a restriction endonuclease recognizing AAGCTT [J. Mol Biol., 92, 331 (1975)]. Separately, 89 nucleotides upstream of the human interferon $\beta_1$ gene [Taniguchi et al., Gene, Vol. 10, pp. 11 - 15 (1980)] is removed using exonuclease Bal 31 [Nucleic Acids Research 5, 1445 - 1463 (1978)], and a Hind III linker and a Bam HI linker are combined thereto. The product is combined downstream from a lac portable promoter which is a promoter fragment of β-galactosidase of Escherichia·coli and has 105 base pairs [References: (1) Taniguchi et al., Proc. Natl. Acad. Sci. USA, Vol. 77, No. 9, pp. 5230 - 5233 (1980), (2) Roberts & Lauer, Methods in Enzymology, Vol. 68, pp. 473 - 481 (1979)] followed by insertion into the plasmid. The thus obtained plasmid is named PIE 208-LR2 (refer to Fig. 2).

The PIE 208-LR2 plasmid lacks a part of the interferon gene downstream of the Hinf I cleavage site which is close downstream to the sole Pst I cleavage site present in the gene [Taniguchi et al., Proc. Natl. Acad. Sci., USA, Vol. 77, No. 9, pp. 5230 - 5233 (1980)]. The lacking interferon gene is, therefore, supplied by the following step.

PIE 208-LR2 is cleaved with Pst I (A↓G) which is a restriction endonuclease recognizing CTGCAG [Nucleic Acids Res., 3, 343 (1976)]. Separately TpIF319-13 DNA [Taniguchi et al., Gene, Vol. 10, pp. 11 - 15 (1980)] is cleaved with Pst I. Both plasmids are ligated with T4 DNA

- 6 -                    0064681

ligase.   The thus obtained recombinant plasmid is named pIFNβ-4 (refer to Fig. 2).

The interferon gene in pIFNβ-4 lacks the DNA coding for 6 amino acids upstream from the DNA necessary for coding mature human interferon $\beta_1$.  Herein, "mature" means that intact interferon protein is included in the gene.  In order to construct the gene coding for a mature interferon protein, the DNA coding for the 6 amino acids is prepared by a conventional chemical synthetic method, for example, the method described in (1) Tetrahedron Let. Vol. 28, pp. 2449 - 2452 (1978) or (2) Nucleic Acids Research, Vol. 8, pp. 5473 - 5489 (1980).  The sequence of the synthesized DNA is as follows:

|  | 1 | 2 | 3 | 4 | 5 | 6 |
|---|---|---|---|---|---|---|
|  | Met | Ser | Tyr | Asn | Leu | Leu |
| pCG· | ATG | AGC | TAC | AAC | TTG | CT |
|  | TAC | TCG | ATG | TTG | AAC | GAGCp |

A plasmid coding for the production of mature human interferon $\beta_1$ is constructed using the synthetic DNA, pTuB1P-5 and pIFNβ-4.

In order to combine the interferon gene downstream from the tufB promoter, pTuB1P-5 is cleaved with Cla I and Hind III.  Then, pIFNβ-4 is cleaved with Hpa II (C↓C) which is a restriction endonuclease recognizing CCGG [Biochemistry 12, 3055 (1973)] or Hap II (C↓C) which is a restriction endonuclease recognizing CCGG [Methods Mol. Biol., 7, 113 (1974)] and Hind III followed by isolating a DNA fragment of about 700 base pairs.  The DNA fragment codes for from the 7th to the final amino acids of mature human interferon $\beta_1$ protein and has a downstream Hind III cleavage site derived from pBR322.

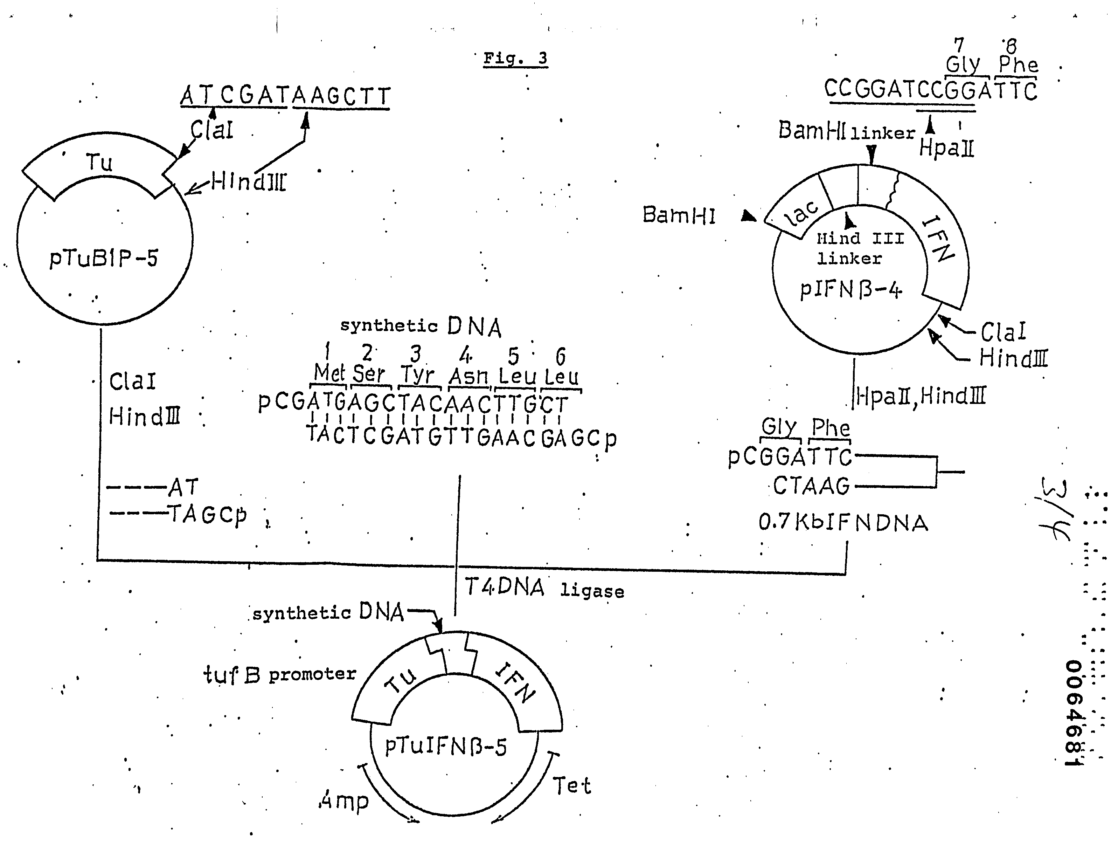

A gene coding for whole protein of mature human interferon β₁ is constructed by ligating the Hap II cleavage site of the DNA fragment and the downstream end of the synthetic DNA mentioned above with T4 DNA ligase as illustrated in Fig. 3. The upstream end of the synthetic DNA and the Cla I cleavage site of pTuBlP-5 which is cleaved with Cla I and Hind III above are ligated with T4 DNA ligase. Thus, the interferon gene is connected with the tufB promoter. The Hind III cleavage site of pTuBlP-5 is ligated to the Hind III cleavage site of the above-mentioned 700 base pair IFN DNA. The constructed plasmid is illustrated in Fig. 3 and named pTuIFNβ-5.

The thus obtained pTuIFNβ-5 plasmid has genes coding for the tufB promoter and whole protein of mature human interferon β₁ as well as genes coding for resistance to ampicillin and tetracycline.

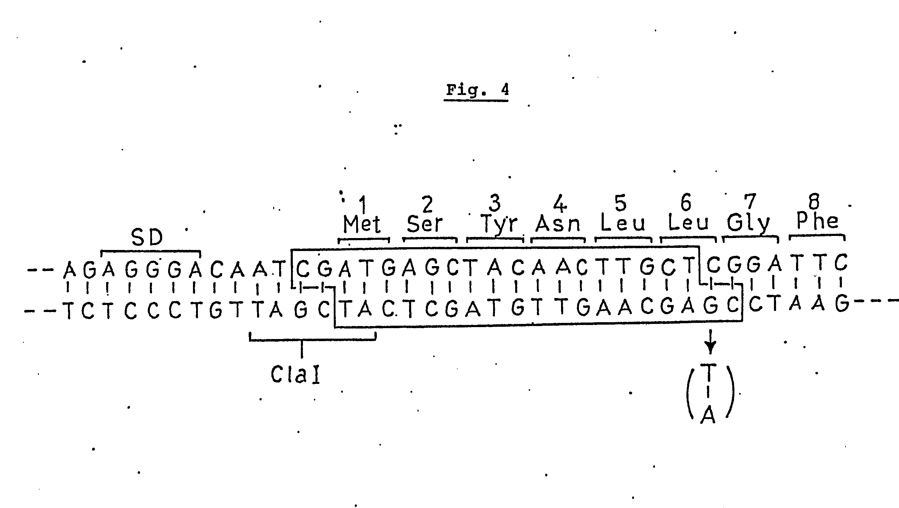

The base sequence of the combining sites of the tufB promoter, synthetic DNA and downstream part of interferon gene is illustrated in Fig. 4. For effective initiation of protein synthesis by the ribosome of Escherichia coli, the presence of a Shine-Dalgarno (SD) sequence rich in purine bases upstream of the ATG codon is necessary and the distance between the SD sequence and ATG codon is important. As illustrated in Fig. 4, the sequence between the SD sequence and ATG codon which codes for the first amino acid of interferon gene in pTuIFNβ-5 is exactly the same as that in the original tufB gene.

The codon used for the 6th amino acid in the human interferon β₁ gene is CTT and in the case of the interferon β₁ gene constructed with the synthetic DNA, the codon is CTC. Since both codons code for leucine, the amino acid sequence of the mature interferon β₁ protein synthesized using pTuIFNβ-5 should be the same as that of natural human protein.

A strain of <u>Escherichia coli</u> is transformed with pTuIFNβ-5 by the method described in Mandel, M. & Higa, A., J. Mol. Biol., <u>53</u>, 159-162 (1970). The thus obtained transformant is cultured using a conventional method for <u>Escherichia coli</u> to determine the productivity of interferon. The productivity is improved in comparison to a transformant containing plasmid pLG117 previously constructed by the present inventors [(Proc. Natl. Acad. Sci., USA, Vol. 77, No. 9, 5230 - 5233 (1980)].

As pTuIFNβ-5 gives an intact interferon gene by cleaving just before the ATG codon with Cla I and the gene can be readily combined with another promoter, pTuIFNβ-5 is very useful. Moreover, it is possible to promote the expression of interferon gene by <u>in vitro</u> modification such as deletion of the tufB promoter in pTuIFNβ-5.

Certain specific embodiments of the invention are illustrated by the following representative examples reflecting actual experimental data.

<u>Example 1</u>
Preparation of plasmid pTuB1P-5:

In this Example, 260 µg of plasmid pTUB1 DNA [Miyajima et al., FEBS Letters, Vol. 102, No. 2, pp. 207 - 210 (1979)] is incubated at 37°C overnight in 500 µl of a solution consisting of 10 mM Tris-HCl (pH 7.5), 6 mM $MgCl_2$, 10 mM NaCl, 6mM mercaptoethanol and 100 units of Bgl II [product of Bethesda Research Laboratories (BRL), USA]. The reaction mixture is heated at 70°C for 3 minutes and dATP, dCTP, dGTP and dTTP are added each at a concentration of 500 nmoles. Then, 10 units of DNA polymerase I (Klenow's fragment) (product of BRL, USA) is added and the mixture is allowed to react at room temperature (23°C) for 3 hours. After treating with phenol, a DNA precipitated with ethanol is incubated at 37°C overnight in 500 μl of a solution consisting of 10 mM Tris-HCl (pH 7.5), 6mM MgCl$_2$, 10 mM NaCl, 6 mM mercaptoethanol, and 100 units of Cla I (product of Boehringer Mannheim GmbH). The thus obtained DNA is subjected to 1% agarose gel electrophoresis and a DNA fragment of about 1,000 base pairs containing the tufB promoter is recovered by Tabak & Flavell's method described in Nucleic Acids Res., Vol. 5, pp. 2321 - 2332 (1978). The fragment has a Bgl II cleavage site constructing blunt end by DNA polymerase at the 5' end of tufB promoter and a Cla I cleavage site constructing cohesive end at the 3' end.

Separately, 20 μg of pBR 322 DNA [Bolivar et al., Gene, Vol. 2, pp. 95 - 113 (1977)] is incubated at 37°C for 60 minutes in 100 μl of a solution consisting of 10 mM Tris-HCl (pH 7.5), 6 mM MgCl$_2$, 100 mM NaCl, 6 mM mercaptoethanol and 20 units of Eco RI (product of Takara Shuzo Co.). Then, the reaction mixture is heated at 70°C for 3 minutes and dATP and dTTP are added each at a concentration of 100 nmoles. Then, 3 units of DNA polymerase I (Klenow's fragment) is added and the mixture is allowed to react at room temperature (23°C) for 3 hours. After treating with phenol, a DNA precipitated with ethanol is incubated at 37°C for 60 minutes in 100 μl, of a solution consisting of 10 mM Tris-HCl (pH 7.5), 6 mM MgCl$_2$, 10 mM NaCl, 6 mM mercaptoethanol and 20 units of Cla I. After treating with phenol and ethanol, the resulting DNA precipitate is dissolved in 50 μl of 10 mM Tris-HCl (pH 7.5).

Then, 1 μg of pBR 322 DNA mentioned above and 0.4 μg of the DNA fragment containing the tufB promoter are incubated at 15°C for 4 hours in 20 μl of a solution consisting of 66 mM Tris-HCl (pH 7.6), 6.6 mM MgCl$_2$, 10mM dithiothreitol, 1 mM ATP and 0.2 unit of T4 DNA ligase (product of Takara Shuzo Co.).

Escherichia coli HB101 strain [J. Mol. Biol., 41, 459 - 472 (1969)] is transformed with the above reaction product by the method described in Mandel & Higa, J. Mol. Biol., 53, 159 - 162 (1970) followed by isolating plasmid pTuB1P-5 from the transformants resistant to ampicillin. The plasmid contains a DNA fragment of about 1,000 base pairs containing the tufB promoter at the Eco RI - Cla I cleavage site of pBR 322 DNA and the tufB promoter is cleaved in the downstream part with Cla I just like the tufB gene.  A flow chart of the foregoing process is illustrated in Fig. 2.

A transformant, Escherichia coli HB101 carrying the plasmid pTuB1P-5, has been deposited with the American Type Culture Collection, U.S.A., as Escherichia coli ATCC 31878.

Example 2

Preparation of plasmid pIFNβ-4:

In this Example, 10 μg of plasmid pTR 56 DNA [Proc. Natl. Acad. Sci. USA, Vol. 77, No. 9, pp. 5230 - 5233 (1980)] is incubated at 37°C for 60 minutes in 100 μl of a solution consisting of 10 mM Tris-HCl (pH 7.5), 6 mM $MgCl_2$, 50 mM NaCl, 6 mM mercaptoethanol, and 10 units of Hind III (product of BRL, USA).  The mixture is treated with phenol to remove proteins and with ethanol to obtain a precipitate.  The precipitate is dissolved in 40 μl of a solution consisting of 12 mM $CaCl_2$, 12 mM $MgCl_2$, 600 mM NaCl, 20 mM Tris-HCl (pH 8.1) and 1mM EDTA and 2 units of Bal 31 (product of BRL, USA) is added.  The mixture is allowed to react at 30°C for 10 minutes.  After treating with phenol and ethanol, the resulting precipitate is dissolved in 100 μl of a solution consisting of 10 mM Tris-HCl (pH 7.5), 6 mM $MgCl_2$, 100 mM NaCl and 6mM mercaptoethanol and 10 units of Bam HI (product of BRL, USA) is added.

The mixture is incubated at 37°C for 60 minutes. After treating with phenol and ethanol, the resulting precipitate is reacted with 20 p.mole Bam HI linker and Hind III linker respectively which are products of Collaborative Research Co. and the 5' end of each DNA is phosphorylated with T-4 polynucleotidekinase and 30 p.mole lac portable promoter which is a promoter fragment of β-galactosidase gene of $\underline{Escherichia}$ $\underline{coli}$ and consisting of 105 base pairs [(1) Taniguchi et al., Proc. Natl. Acad. Sci. USA, Vol. 77, No. 9, pp. 5230 - 5233 (1980), (2) Roberts & Lauer, Methods in Enzymology, Vol. 68, pp. 473 - 481 (1979)] together with 1 unit of T4 DNA ligase (product of BRL, USA) in 50 μl of a solution consisting of 66 mM Tris-HCl (pH 7.6), 6.6 mM $MgCl_2$, 10mM dithiothreitol, 1mM ATP by incubating at 20°C for 4 hours.

$\underline{Escherichia}$ $\underline{coli}$ LG90 strain (Guarente et al., Cell, Vol. 20, pp. 543 - 553) is transformed with the reaction product according to the method described in Mandel & Higa, J. Mol. Biol., $\underline{53}$, 159-162 (1970). Plasmid PIE 208-LR2 is isolated from ampicillin resistant transformants. Compared with plasmid pTR 56, plasmid PIE 208-LR2 has a Bam HI linker inserted next to the 90th A-T base pair of interferon β cDNA [TpIF319-13, Taniguchi et al., Gene Vol. 10, pp. 11 - 15 (1980)]. Then, 1 μg of the plasmid DNA is incubated at 37°C for 60 minutes in 20 μl of a solution consisting of 10 mM Tris-HCl (pH 7.5), 6mM $MgCl_2$, 50 mM NaCl, 6 mM mercaptoethanol and 1 unit of Pst I (product of Takara Shuzo Co.).

Separately, 1 μg of plasmid TpIF 319-13 DNA is cleaved with Pst I as mentioned above and both reaction solutions are mixed. After treating with phenol, the DNA precipitated with ethanol is reacted with 0.2 unit of T4 DNA ligase (product of Takara Shuzo Co.) in 25 μl of a solution consisting of 66 mM Tris-HCl (pH 7.5), 6.6 mM

MgCl$_2$, 10 mM dithiothreitol and lmM ATP at 15°C for 4 hours. Escherichia coli HB101 strain is transformed with the reaction product. Plasmid pIFNβ-4 is isolated from an ampicillin and tetracycline resistant transformant. The foregoing process is also illustrated in Fig. 2.

Example 3
Preparation of plasmid pTuIFNβ-5:

In this Example, 30 μg of pTuBlP-5 DNA is incubated at 37°C for 60 minutes in 60 μl of a solution consisting of 10 mM Tris-HCl (pH 7.5), 6mM MgCl$_2$, 6mM mercaptoethanol and 30 units of Cla I. To the reaction solution, 50 mM NaCl is added to make up 100 μl of a solution consisting of 10 mM Tris-HCl (pH 7.5), 6 mM MgCl$_2$, 50 mM NaCl, and 6mM mercaptoethanol. Then, 30 units of Hind III (product of Takara Shuzo Co.) is added to the solution and the mixture is incubated at 37°C for 60 minutes. After treating with phenol and ethanol, the resulting DNA precipitate is dissolved in 10 μl of 10 mM Tris-HCl (pH 7.5). The solution contains 1.155 μg/μl DNA.

Then, 400 μg of pIFNβ-4 DNA is incubated at 37°C overnight in 1.7 ml of a solution consisting of 10 mM Tris-HCl (pH 7.5), 6 mM MgCl$_2$, 6 mM mercaptoethanol and 100 units of Hap II (product of Takara Shuzo Co.). To the reaction solution 50 mM NaCl is added to make up 1.8 ml of a solution consisting of 10 mM Tris-HCl (pH 7.5), 6 mM MgCl$_2$, 50 mM NaCl and 6 mM mercaptoethanol. Then, 128 units of Hind III is added and the mixture is incubated at 37°C overnight. The thus treated DNA is subjected to 1% agarose gel electrophoresis and a fragment containing interferon β$_1$ cDNA of about 700 base pairs is recovered by the method described in Tabak and Flavell, Nucleic Acids Res. Vol. 5, pp. 2321 - 2332 (1978). 5 μg of DNA is recovered from the fragment. The DNA has a sequence coding for all amino acids from the 7th amino acid, glycine, to the end of interferon $\beta_1$ and has a Hap II cleavage site at the 5' sticky end and a Hind III cleavage site at the 3' sticky end. (refer to Fig. 3)

Two DNAs having the following sequences are synthesized according to the methods described in (1) Hirose et al.; Tetrahedron Let., Vol. 28, pp. 2449 - 2452 (1978), or (2) Miyoshi et al.; Nucl. Acids Res., Vol. 8, pp. 5473 - 5489 (1980).

1.   (5') C-G-A-T-G-A-G-C-T-A-C-A-A-C-T-T-G-C-T (3')
2.   (5') C-G-A-G-C-A-A-G-T-T-G-T-A-G-C-T-C-A-T (3')

Thereafter, 87 p.moles each of the DNAs are incubated at 30°C for 20 minutes in 20 µl of a solution consisting of 50 mM Tris-HCl (pH 7.6), 10 mM $MgCl_2$, 5 mM mercaptoethanol, 0.3 mM ATP and 3.3 units of T-4 polynucleotidekinase (product of Boehringer Mannheim GmbH) to phosphorylate the 5' end.

Then, 13 p.moles of the thus chemically synthesized DNAs are reacted with 1 µg of the Hap II-Hind III DNA fragment containing 0.7 µg of pTuB1P-5 DNA cleaved with Cla I and Hind III and interferon $\beta_1$ cDNA together with 0.2 unit of T4 DNA ligase (product of Takara Shuzo Co.) in 30 µl of a solution consisting of 66 mM Tris-HCl (pH 7.6), 6.6 mM $MgCl_2$, 10 mM dithiothreitol and 1 mM ATP at 15°C for 15 hours. Escherichia coli HB101 strain is transformed with the reaction product and plasmid pTuIFNβ-5, illustrated in Fig. 3, is isolated from an ampicillin resistant transformant. The foregoing process is also diagramatically illustrated in Fig. 3. The base sequence of the combining site of the tufB promoter, synthetic DNA and interferon $\beta_1$ is illustrated in Fig. 4.

The transformant of _Escherichia coli_ HB101 with pTuIFNβ-5
has been deposited with the American Type Culture
Collection, U.S.A., under accession number ATCC 31879.

## Example 4

Determination of interferon activity:

In this example, _Escherichia coli_ HB101 strains carrying
pTuIFNβ-5 and pLG 117-R [Taniguchi et al., Proc. Natl.
Acad. Sci. USA, Vol. 77, No. 9, pp. 5230 - 5233 (1980)]
are cultured with stirring at 37°C in 100 ml of 25 µg/ml
ampicillin-containing L-broth consisting of 1%
bactotrypton (DIFCO), 0.5% yeast extract (DIFCO) and 0.5%
NaCl to make up $1 - 2.5 \times 10^7$ cells/ml culture broth.
The culture broth is subjected to centrifugation at 5,000
rpm for 5 minutes and the resulting precipitate is
suspended in 1 ml of 50 mM Tris-HCl (pH 8.0) containing
25% sucrose at 0°C. Then, 0.2 ml of 5 mg/ml lysozyme
(product of Sigma Co., USA) is added under ice cooling and
the mixture is allowed to stand for 5 minutes. Then, 0.2
ml of 0.25 M $Na_2$-EDTA (pH 8.0) is added and the mixture
is allowed to stand for 5 minutes, after which, 0.3 ml of a
solution consisting of 5% Brij 58 (product of Wako Junyaku
Co.), 2% sodium 7-deoxycholic acid, 0.3 M $Na_2$-EDTA and
0.25 M Tris-HCl (pH 8.0) is added. The mixture is allowed
to stand under ice cooling for 10 minutes. After
centrifugation at 40,000 rpm for 30 minutes, about 1.8 ml
of a supernatant is obtained. Interferon activity of the
supernatant is determined according to the CPE-reading
method [Finter, N.B., J. Gen. Virol., Vol. 5, pp. 419 -
425 (1969)]. For the measurement, as human cells, GM 258
available from Human Genetic Mutant Cell Repository,
Camden, N.J., U.S.A., and as challenge virus,
encephalomyocardits (EMC) virus available from the NIH,
USA, are used. Interferon activity of the extract of

<u>Escherichia</u> <u>coli</u> cells are determined using interferon β available from the NIH as a control. The average of three measurements is as follows:

(1) pTuIFNβ-5/HB101 : 1,700 units/ml
(2) pLG117R/HB101 : 400 units/ml

In the case of (1), $1.0 \times 10^{8}$ cells have a total interferon activity of about 3,000 units and in the case of (2), $2.5 \times 10^{8}$ cells have a total interferon activity of about 700 units. Therefore, it will be appreciated that the HB101 strain carrying pTuIFNβ-5 produces about 10 times more interferon β than that of pLG117R.

WHAT IS CLAIMED IS:

1.   A recombinant DNA containing the tufB promoter of
Escherichia coli.

2.   The recombinant DNA according to claim 1 which is a
recombinant with a plasmid DNA.

3.   The recombinant DNA according to claim 2, wherein said
plasmid DNA is derived from Escherichia coli.

4.   A recombinant DNA wherein a gene coding for the
biosynthesis of eukaryotic protein is combined downstream
of the tufB promoter of Escherichia coli.

5.   The recombinant DNA according to claim 4 which is a
recombinant with a plasmid DNA.

6.   The recombinant DNA according to claim 5, wherein said
plasmid DNA is derived from Escherichia coli.

7.   The recombinant DNA according to claim 4, wherein said
gene coding for the biosynthesis of eukaryotic protein is
human interferon $\beta_1$ gene.

8.   A recombinant DNA containing a gene coding for the
biosynthesis of eukaryotic protein, wherein a cleavage
site for restriction endonuclease Cla I is constructed
upstream from the gene.

9.   A process for producing eukaryotic protein, which comprises culturing a microorganism carrying a plasmid containing the tufB promoter of <u>Escherichia</u> <u>coli</u> and a gene coding for eukaryotic protein and which is capable of producing said eukaryotic protein in a nutrient medium, until recoverable quantities of said protein are accumulated in the culture liquor and thereafter isolating said protein therefrom.

10.   The process according to claim 9, wherein said eukaryotic protein is human interferon $\beta_1$.

11.   The process according to claim 9, wherein said microorganism belongs to <u>Escherichia</u> <u>coli</u>.

12.   The process according to claim 9, wherein said plasmid is pTuIFN β-5.

13.   <u>Escherichia</u> <u>coli</u> carrying plasmid pTuBlP-5.

14.   <u>Escherichia</u> <u>coli</u> carrying plasmid pTuIFNβ-5.

Fig. 1

0064681

2/4

Fig. 2

0064681

Fig. 3

ATCGATAAGCTT

ClaI

HindIII

Tu

pTuBIP-5

ClaI
HindIII

----AT
----TAGCp

synthetic DNA

| 1 | 2 | 3 | 4 | 5 | 6 |
|---|---|---|---|---|---|
| Met | Ser | Tyr | Asn | Leu | Leu |

pCGATGAGCTACAACTTGCT
TACTCGATGTTGAACGAGCp

7   8
Gly Phe
CCGGATCCGGATTC

BamHI linker     HpaII

BamHI

lac

Hind III
linker

IFN

pIFNβ-4

ClaI
HindIII

HpaII, HindIII

Gly Phe
pCGGATTC
CTAAG

0.7 Kb IFN DNA

T4 DNA ligase

synthetic DNA

tuf B promoter

Tu     IFN

pTuIFNβ-5

Amp     Tet

## Fig. 4

```
                                    1     2     3     4     5     6     7     8
                                   Met   Ser   Tyr   Asn   Leu   Leu   Gly   Phe
              SD
--AGAGGGACAAT CGATGAGCTACAACTTGCT CGGATTC
  | | | | | | | | | | | |  | | | | | | | | | | | | | | | | | | |  | | | | |
--TCTCCCTGTTAGC TACTCGATGTTGAACGAGC CTAAG---

          ClaI                                            ↓
                                                        ( T )
                                                        ( | )
                                                        ( A )
```